# EUROPEAN PATENT APPLICATION

(11) **EP 4 458 347 A1**
(43) Date of publication of application: **06.11.2024**
(21) Application number: 23743429.5
(22) Date of filing: 16.01.2023
(51) Int. Cl.: A61K 9/00, A61K 47/02, A61K 47/26, A61K 31/4725, A61K 31/422, A61P 19/00

(54) **INJECTABLE FORMULATION CONTAINING ISOXAZOLINE DERIVATIVE**

(30) Priority: 24.01.2022 KR 20220010141
(71) Applicant: LG Chem, Ltd., Yeongdeungpo-gu Seoul 07336 (KR)
(72) Inventor: BAEK, Jaeuk, Daejeon 34122 (KR); KIM, Sung Won, Daejeon 34122 (KR); YOON, Jung Woon, Daejeon 34122 (KR)
(74) Representative: Hoffmann Eitle
(86) International application number: PCT/KR2023/000757
(87) International publication number: WO 2023/140582

(57) **Abstract**

The present invention relates to an injectable formulation containing, as an active pharmaceutical ingredient (API), an isoxazoline derivative or pharmaceutically acceptable salt thereof useful as a caspase inhibitor. The injectable formulation according to the present invention includes a first agent composed of a high dose of the API, and a second agent that is a solvent capable of dissolving the first agent, and may be prepared by mixing the first agent and the second agent immediately before administration. The injectable formulation stably contains the active API and thus has the advantage that effective drug efficacy can be expected when administered to a patient.

## Description

### [Technical Field]

This application claims the benefit of priority based on Korean Patent Application No. 10-2022-0010141 filed on January 24, 2022, all contents of which are incorporated herein as part of this specification.

The present invention relates to a preparation for injection comprising an isoxazoline derivative useful as a caspase inhibitor or a pharmaceutically acceptable salt thereof as an active pharmaceutical ingredient (API).

### [Background Art]

Caspase is a cysteine protease, and a caspase inhibitor is a compound that can control inflammation or apoptosis caused by the action of caspase by inhibiting the action of this caspase. Diseases for which this compound can be administered to eliminate or alleviate symptoms include osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes, gastric ulcer and the like.

As caspase inhibitors, various types of isoxazoline derivatives and prodrugs of the isoxazoline derivatives are known. Among the isoxazoline derivatives, (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide(Hereinafter, referred to as "Compound of Formula 1") has already proven its efficacy in liver disease caused by hepatitis virus, liver fibrosis, and nonalcoholic steatohepatitis (NASH).

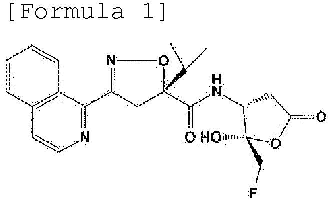

Recently, attempts have been made to apply the compound of Formula 1 to diseases related to bone and joint, such as osteoarthritis (OA), rheumatoid arthritis, degenerative arthritis, and destructive bone disorders.

In the treatment of the diseases related to bone and joint, a common drug administration method is to inject directly with a syringe into the joint cavity filled with synovial fluid. In the case of oral administration, the drug has a systemic effect, but in the case of direct administration into the joint cavity, the effect of the drug is local, so it is possible to administer a high concentration of the drug and minimize systemic side effects.

The compound of Formula 1 has been confirmed to have a therapeutic effect as the existing oral preparation for liver disease, but since the compound of Formula 1 is poorly soluble in water, it is difficult to formulate it in a preparation for injection.

Therefore, it is necessary to develop a high-concentration preparation for injection that can use the compound of Formula 1 as an injection.

### [Prior Art Documents]

### [Patent Document]

(Patent Document 1) Korean Patent No. 0774999(2007.11.02.) Isoxazoline derivatives and novel process for its preparation
(Patent Document 2) Korean Patent Application No. 2021-0102476(2021.08.04.) Use of caspase inhibitor for alleviation or treatment of osteoarthritis

### [Non-Patent Document]

(Non-Patent Document 1) Ratziu et al., 'A Phase 2, Randomized, Double-Blind, Placebo-Controlled Study of GS-9450 in Subjects with Nonalcoholic Steatohepatitis' HEPATOLOGY, Vol. 55, No. 2, 2012.

### [Disclosure]

### [Technical Problem]

Accordingly, the inventors of the present invention have conducted various studies to solve the above problems, and as a result, the problem to be solved by the present invention is to derive a preparation for injection comprising a high content of the compound of Formula 1 as an active pharmaceutical ingredient (API).

### [Technical Solution]

The present invention provides a preparation for injection comprising the first formulation containing the compound of Formula 1; and the second formulation, which is an aqueous solution with a pH of 7 to 11 containing a phosphate buffer to dissolve the compound of Formula 1, surfactant, and NaOH as a pH adjusting agent.

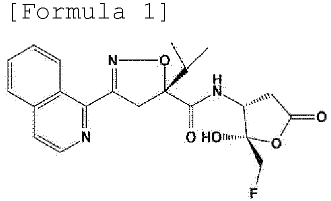

The content of the compound of Formula 1 contained in the first formulation of the preparation for injection of the present invention is 5 to 20 mg/ml based on a mixed solution of the first formulation and the second formulation.

The contents of the phosphate buffer, surfactant, NaOH contained in the second formulation of the preparation for injection of the present invention are 100 to 130 mM, 0.2 to 5.0(w/v)% and 12 to 42 mM based on the second formulation, respectively.

The preparation for injection of the present invention is used to treat or prevent a caspase-related disease selected from osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

The preparation for injection of the present invention is used within 30 minutes after mixing the first formulation and the second formulation, immediately before administration to the patient.

### [Advantageous Effects]

The preparation for injection comprising the compound of Formula 1 according to the present invention includes the first formulation in powder form containing the compound of Formula 1; and the second formulation as a buffer for dissolving the first formulation. Since the preparation for injection of the present invention is prepared by mixing the first formulation in powder form and the second formulation acting as a agent for injection right before administration to a patient, and administer to the patient within 30 minutes, it has an advantage of high active-API content.

### [Description of Drawings]

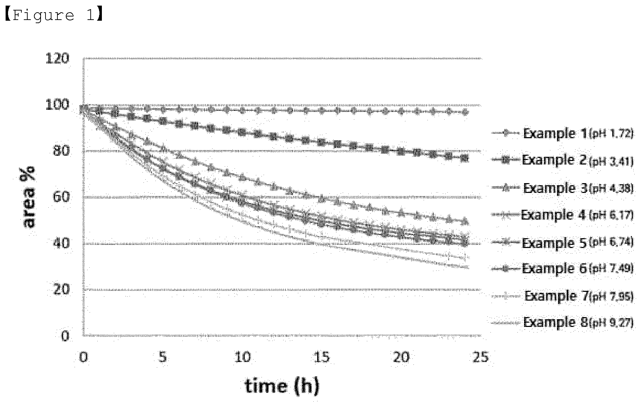

Fig. 1 shows the content of active-API according to the pH of the solvent.

### [Best Mode]

Hereinafter, the present invention will be described in detail.

All technical terms used in the present invention, unless defined otherwise, are used in the same meaning as commonly understood by those of ordinary skill in the art related to the present invention. In addition, although preferred methods or samples are described in this specification, those similar or equivalent thereto are also included in the scope of the present invention. The contents of all publications incorporated by reference herein are hereby incorporated by reference in their entirety.

The present inventors continued research in various ways to develop a preparation for injection containing (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide of Formula 1, which is insoluble in water, as an API. As a result, the present inventors have developed a preparation for injection comprising the first formulation containing the API; and the second formulation, which is an aqueous solution containing a phosphate buffer, surfactant, and a pH adjusting agent.

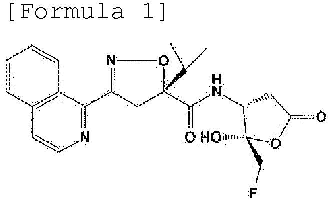

In one embodiment of the present invention, the pH adjusting agent is NaOH, and pH of the second formulation has a pH of 7 to 11.

In one embodiment of the present invention, in the preparation for injection, the first formulation is in the powder form.

In one embodiment of the present invention, the compound of Formula 1 is contained in an amount of 5 to 20 mg/ml based on a total volume of a mixed solution of the first formulation and the second formulation.

In one embodiment of the present invention, a mixed solution of the first formulation and the second formulation has a pH of 6 to 8.

In one embodiment of the present invention, a mixed solution of the first formulation and the second formulation has an osmotic pressure of 280 to 330 mOsm/kg.

In one embodiment of the present invention, the phosphate buffer is a mixture of sodium phosphate dibasic heptahydrate and sodium phosphate monobasic monohydrate.

In one embodiment of the present invention, the surfactant is selected from polysorbate 20, polysorbate 40, polysorbate 60, polysorbate 65 and polysorbate 80.

In one embodiment of the present invention, the surfactant is polysorbate 80.

In one embodiment of the present invention, the surfactant is contained in an amount of 0.2 to 5.0(w/v)% based on a total volume of the second formulation.

In one embodiment of the present invention, the NaOH is contained in an amount of 12 to 42 mM based on a total volume of the second formulation.

In one embodiment of the present invention, the preparation for injection is for treating or preventing a disease selected from osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

In one embodiment of the present invention, the disease that the preparation for injection can treat or prevent is selected from osteoarthritis, rheumatoid arthritis, degenerative arthritis and destructive bone disorder.

In one embodiment of the present invention, a mixed solution of the first formulation and the second formulation is for a single-dose injection.

In one embodiment of the present invention, the preparation for injection is administered into the joint cavity after the first formulation and the second formulation are mixed.

In one embodiment of the present invention, the preparation for injection is administered into the joint cavity within 30 minutes after the first formulation and the second formulation are mixed.

In one embodiment of the present invention, the preparation for injection is administered once after the first formulation and the second formulation are mixed.

In one embodiment of the present invention, the preparation for injection is a preparation for injection in which the content of the following compound of Formula 2 is 95% or more of the total content of the compound of Formula 2 and the following compound of Formula 3 when the contents of the compounds of Formula 2 and Formula 3 were measured within 30 minutes after mixing the first formulation and the second formulation.

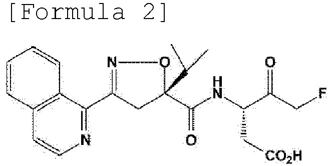

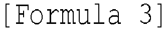

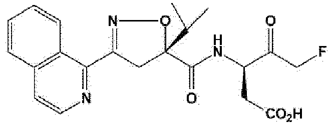

In one embodiment of the present invention, the preparation for injection is a preparation for injection comprising: the first formulation containing the compound of Formula 1 and the second formulation, which is a pH of 7 to 11 buffer to dissolve the compound of Formula 1, wherein, when the contents of the following compounds of Formula 2 and Formula 3 were measured within 30 minutes after mixing the first formulation and the second formulation, of the total contents of the compounds of Formula 2 and Formula 3, the content of the compound Formula 2 is 95% or more.

Since the preparation for injection of the present invention is prepared by mixing the first formulation in powder form and the second formulation acting as a agent for injection right before administration to a patient, and administer to the patient within 30 minutes, it has an advantage of high active-API content.

Since the compound of Formula 1 of the present invention is hardly soluble in water, the solubility was analyzed using buffers of various pH in order to find a solvent capable of dissolving the compound of Formula 1 in high content.

As a result, it was confirmed that the compound of Formula 1 was hardly soluble in acidic solvents with a pH of 4 or less, but began to dissolve in weakly acidic solvents with a pH of 6 or more, and was well soluble in basic solvents with a pH of 7.5 or more. In addition, it was confirmed that the compound of Formula 1 has a property of reducing the pH of a solution while being dissolved in a solvent.

Further, it was found that when the compound of Formula 1 was dissolved in an aqueous solvent, the lactone ring was broken and converted to the compound of Formula 2 below. It was found that at this time, the compound of Formula 2 has an active form, but the compound of Formula 2 is rapidly and irreversibly converted into a structural isomer, the compound of Formula 3, in an aqueous solution, and the compound of Formula 3 is an inactive form that does not show caspase inhibitory activity unlike the compound of Formula 2.

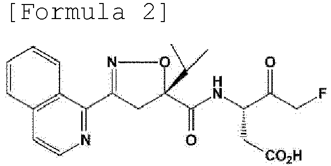

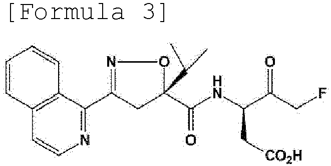

In addition, as a result of measuring the conversion rate from the compound of Formula 2 into the compound of Formula 3 according to the pH of the solvent, it was confirmed that the compound of Formula 3 was hardly produced in an acidic solvent, but the compound of Formula 3 was rapidly produced in a basic solvent with a neutral pH or higher.

Therefore, it was found that, in order to dissolve the compound of Formula 1, a basic solvent with a high pH must be used, but, as the compound of Formula 1 is dissolved, the solubility of the undissolved compound is lowered by lowering the pH of the solution, and the active form of the dissolved compound is rapidly changed to the inactive form in a basic solvent. This mechanism lowers the purity of the active ingredient in the preparation for injection using the compound of Formula 1 as an API, so it may affect the efficacy, and also since this mechanism lowers the pH of the solution as the compound of Formula 1 is dissolved, it is difficult to adjust the pH when preparing the final drug for administration to a subject.

To solve this pH control problem, the preparation for injection of the present invention includes a buffer in the second formulation to dissolve the first formulation containing the API.

As the buffer included in the second formulation, a conventional buffer that can be used for an injection solution may be used, and specific examples include, but are not limited to, phosphate buffer, Tris buffer, and MES buffer. When using a phosphate buffer, it can be selected from the group consisting of sodium phosphate dibasic acid, sodium phosphate monobasic acid, potassium phosphate monobasic acid, potassium phosphate dibasic acid, and mixtures thereof. When using Tris buffer, TRIZMA^{®} buffer can be used.

The buffer has a concentration of 100 to 130 mM based on the second formulation, specifically, 100 to 130 mM, 110 to 130 mM, 120 to 130 mM, 100 to 120 mM, 110 to 120 mM, 100 to 110 mM, 110 to 120 mM, or 120 to 130 mM.

In the preparation for injection of the present invention, the second formulation uses NaOH as a pH adjusting agent.

The NaOH has a concentration of 12 to 42 mM based on the second formulation.

In the preparation for injection of the present invention, the pH of the second formulation is adjusted to pH 7 to 11 using a buffer and a pH adjusting agent, specifically, to 7 to 11, 7.5 to 11, 8 to 11, 8.5 to 11, 9 to 11, 9.5 to 11, 10 to 11, 10.5 to 11, 7 to 10.5, 7.5 to 10.5, 8 to 10.5, 8.5 to 10.5, 9 to 10.5, 9.5 to 10.5, 10 to 10.5, 7 to 10, 7.5 to 10, 8 to 10, 8.5 to 10, 9 to 10, 9.5 to 10, 7 to 9.5, 7.5 to 9.5, 8 to 9.5, 8.5 to 9.5, 9 to 9.5, 7 to 9, 7.5 to 9, 8 to 9, 8.5 to 9, 7 to 8.5, 7.5 to 8.5, 8 to 8.5, 7 to 8, or 7.5 to 8.

In the preparation for injection of the present invention, the second formulation contains surfactant. The surfactant can help dissolve the compound of Formula 1. As the surfactant, a conventional surfactant that can be used for an injection solvent may be used, and a cyclodextrin-based or polysorbate-based surfactant may be used. Specific examples of polysorbate-based surfactants include polysorbate 20, polysorbate 40, polysorbate 60, polysorbate 65, or polysorbate 80.

The concentration of the surfactant may be 0.2 to 5.0(w/v)% based on the second formulation, specifically, 0.2 to 5.0(w/v)%, 0.8 to 5.0(w/v)%, 1.2 to 5.0(w/v)%, 1.6 to 5.0(w/v)%, 2.0 to 5.0(w/v)%, 2.4 to 5.0(w/v)%, 2.8 to 5.0(w/v)%, 3.2 to 5.0(w/v)%, 3.6 to 5.0(w/v)%, 4.0 to 5.0 (w/v)%, 4.4 to 5.0 (w/v)%, 0.2 to 4.4(w/v)%, 0.8 to 4.4(w/v)%, 1.2 to 4.4(w/v)%, 1.6 to 4.4(w/v)%, 2.0 to 4.4(w/v)%, 2.4 to 4.4(w/v)%, 2.8 to 4.4(w/v)%, 3.2 to 4.4(w/v)%, 3.6 to 4.4 (w/v)%, 4.0 to 4.4 (w/v)%, 0.2 to 4.0(w/v)%, 0.8 to 4.0(w/v)%, 1.2 to 4.0(w/v)%, 1.6 to 4.0(w/v)%, 2.0 to 4.0(w/v)%, 2.4 to 4.0(w/v)%, 2.8 to 4.0(w/v)%, 3.2 to 4.0(w/v)%, 3.6 to 4.0(w/v)%, 0.2 to 3.6(w/v)%, 0.8 to 3.6(w/v)%, 1.2 to 3.6(w/v)%, 1.6 to 3.6(w/v)%, 2.0 to 3.6(w/v)%, 2.4 to 3.6(w/v)%, 2.8 to 3.6(w/v)%, 3.2 to 3.6(w/v)%, 0.2 to 3.2(w/v)%, 0.8 to 3.2(w/v)%, 1.2 to 3.2(w/v)%, 1.6 to 3.2(w/v)%, 2.0 to 3.2(w/v)%, 2.4 to 3.2(w/v)%, 2.8 to 3.2(w/v)%, 0.2 to 2.8(w/v)%, 0.8 to 2.8(w/v)%, 1.2 to 2.8(w/v)%, 1.6 to 2.8(w/v)%, 2.0 to 2.8(w/v)%, 2.4 to 2.8(w/v)%, 0.2 to 2.4(w/v)%, 0.8 to 2.4(w/v)%, 1.2 to 2.4(w/v)%, 1.6 to 2.4(w/v)%, 2.0 to 2.4(w/v)%, 0.2 to 2.0(w/v)%, 0.8 to 2.0(w/v)%, 1.2 to 2.0(w/v)%, 1.6 to 2.0(w/v)%, 0.2 to 1.6(w/v)%, 0.8 to 1.6(w/v)%, 1.2 to 1.6(w/v)%, 0.2 to 1.2(w/v)%, 0.8 to 1.2(w/v)%, or 0.2 to 0.8(w/v)%.

In the preparation for injection of the present invention, when the first formulation and the second formulation are mixed for administration to a patient, as the compound of Formula 1 contained in the first formulation dissolves in the second formulation, the pH of the solution is lowered. If the pH of the mixed solution of the first formulation and the second formulation is too low, it may cause pain to the patient, so the pH of the mixed solution of the first formulation and the second formulation should be between 6 and 8.

Specifically, the pH of the mixed solution of the first formulation and the second formulation may be 6 to 8, 6 to 7.7, 6 to 7.4, 6 to 7.1, 6 to 6.8, 6 to 6.5, 6 to 6.2, 6.3 to 8, 6.3 to 7.7, 6.3 to 7.4, 6.3 to 7.1, 6.3 to 6.8, 6.3 to 6.5, 6.6 to 8, 6.6 to 7.7, 6.6 to 7.4, 6.6 to 7.1, 6.6 to 6.8, 6.9 to 8, 6.9 to 7.7, 6.9 to 7.4, 6.9 to 7.1, 7.2 to 8, 7.2 to 7.7, 7.2 to 7.4, 7.5 to 8, or 7.5 to 7.7.

In the preparation for injection of the present invention, the osmotic pressure of the mixed solution of the first formulation and the second formulation is 280 to 330 mOsm/kg. If the osmotic pressure is lower or higher than the above range, it is undesirable because it may cause pain to the patient.

In the preparation for injection of the present invention, the content of the compound of Formula 1 has a concentration of 5 to 20 mg/ml based on the mixture of the first formulation and the second formulation.

Specifically, the content of the compound of Formula 1 may be 5 to 20 mg/ml, 10 to 20 mg/ml, 15 to 20 mg/ml, 5 to 15 mg/ml, 10 to 15 mg/ml, 5 to 10 mg/ml, 5 mg/ml, 10 mg/ml, 15 mg/ml, or 20 mg/ml based on the mixture of the first formulation and the second formulation, preferably in single dose.

In the preparation for injection of the present invention, when the compound of Formula 1 included in the first formulation is mixed with the second formulation, the compound is completely dissolved, and the mixed solution of the first formulation and the second formulation is in a transparent state.

The preparation for injection of the present invention is used by mixing the first formulation and the second formulation immediately before administration to the patient, so it does not additionally contain excipients for preservation such as antioxidants and preservatives.

The present invention provides a method for treating or preventing caspase-related diseases using the preparation for injection of the present invention.

The term "treating" means stopping or delaying the progression of the disease when used in a subject showing symptoms of disease, and the "preventing" means stopping or delaying the signs of a disease when used in a high-risk subject who does not show symptoms of disease.

The "caspase-related disease" of the present invention refers to a disease that can be treated or prevented by inhibiting caspase, for example, osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer, but is not limited thereto the above disease.

The preparation for injection of the present invention can be administered to a subject in need of treatment or prevention of a caspase-associated disease to treat or prevent the disease in the subject.

The term "administration" or "administering" as used herein refers to a method of administering by injection a dose of the preparation for injection of the present invention to a vertebrate including mammals, birds, fish or amphibians or non-vertebrate animal. Preferred administration methods may be selected from conventional injection methods such as intravascular injection, subcutaneous injection, transdermal injection, intramuscular injection, spinal injection, and intradermal injection, depending on the type of disease to be treated, the site of the disease, and the severity of the disease. In particular, injection into the joint cavity can be selected for diseases related to bone joints such as osteoarthritis, rheumatoid arthritis, degenerative arthritis, or destructive bone disorders.

In the case of injection into the joint cavity using the preparation for injection of the present invention, administration may be guided using an imaging method using ultrasound or the like, or a local anesthetic may be administered in advance or in combination.

The term "subject" as used herein refers to a human or non-human mammal, such as a dog, cat, mouse, rat, cow, sheep, pig, goat, non-human ape, or bird. In some embodiments, the subject is a human.

The preparation for injection of the present invention is administered to the subject within 30 minutes after making a mixed solution by mixing the first formulation and the second formulation. Therefore, the preparation for injection of the present invention is intended for single-dose for singe patient infusion or administration.

In addition, the preparation for injection of the present invention may be additionally administered in combination with drugs such as analgesics, anti-inflammatory drugs, and steroids. Specifically, it can be administered in combination with drugs such as non-steroidal anti-inflammatory drugs (NSAIDs) selected from ibuprofen, naproxen, aspirin, acetaminophen, indomethacin, diclofenac taken orally or at the site of the lesion, meloxicam, celecoxib, piroxicam, etodolac, nabumetone, lumiracoxib, valdecoxib, etoricoxib, parecoxib, fenoprofen, oxaprozin, mefenamic acid, diflunisal, fluvirofen and ketoprofen; corticoid drugs such as prednisone, methylprednisolone; narcotic pain relievers such as codeine, fentanyl, morphine and meperidine; or injection of hyaluronic acid derivative.

The present invention provides a kit comprising the preparation for injection of the present invention. In one embodiment of the present invention, the kit may include a preparation for injection comprising the first formulation containing the compound of Formula 1 of the present invention, and the second formulation which is an aqueous solution for dissolving the compound of Formula 1; an administration system such as one or more syringes for administering the agent; and instructions for using the kit, including how to mix the first formulation and the second formulation, and directions for treating patients.

In one embodiment of the present invention, the kit may include a label stating that the formulation and contents as described herein are administered to a patient suffering from a disease associated with bone joints such as osteoarthritis, rheumatoid arthritis, degenerative arthritis and destructive bone disorder. Herein, selecting a dosage suitable for the patient is within the knowledge of a person skilled in the art in consideration of the patient's symptoms, age, etc.

Unless otherwise indicated, all numbers used in the specification and claims are to be understood as being modified in all instances by the term "about," whether or not so stated. It should also be understood that the precise numerical values used in the specification and claims form additional embodiments of the disclosure. Efforts have been made to ensure the accuracy of the numerical values disclosed in the Examples. Any measured numerical value, however, can inherently contain certain errors resulting from the standard deviation found in its respective measuring technique.

Various evaluations in Examples and Comparative Examples were conducted as follow.

### Preparation Example

(R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide, a compound of Formula 1 used as API in the present invention (Hereinafter referred to as "API") was prepared according to KR 10-0774999.

### Test Example 1: Confirmation of API solubility according to solvent pH (Example 1 to 10)

In order to derive the optimal pH to dissolve the API, the API was dissolved as much as possible for 24 hours using 10 ml each of sodium phosphate buffer (PB) having various pH (Examples 1 to 9) and distilled water (Example 10). Then, the pH and concentrations of an active substance (Compound of Formula 2) and an inactive substance (Compound of Formula 3) were analyzed using HPLC.

As a result, the API was more soluble in basic solvents (Example 8 and 9), and more soluble in phosphate buffer (Example 4) than in distilled water (Example 10) even at a similar pH. In addition, it was confirmed that acidic solvents did not dissolve the API well compared to neutral or basic solvents, but conversion from the active substance to the inactive substance was lower than that of basic solvents (Example 1).

In addition, after dissolving the API in solvents, the change in the content of the active compound of Formula 2 was measured over time. As a result, in the case of Example 1, the content of the active compound is maintained until 25 hours after dissolution, but the content of the active compound decreases rapidly as the pH increases. In particular, Example 3 to Example 8 show that 20 to 30% of the active compound is converted into the inactive compound within 5 hours (Fig. 1).

Therefore, it can be seen that although the API is well soluble in basic solvents, the conversion rate from the active compound of Formula 2 into the inactive compound of Formula 3 after dissolution is higher in basic solvents than in acidic solvents. In addition, it can be seen that the pH of the solution is lower than the pH of the solvent in all of the solvents of Examples 1 to 10, and especially in the case of Example 10, since distilled water has no buffering effect, the pH rapidly decreases.

**[Table 1]**

| Example | Solvent pH | Solution pH | Active+Inactive Conc. (mg/ml) | Active Conc. (mg/ml) | Inactive Conc. (mg/ml) |
|---|---|---|---|---|---|
| Example 1 | 1.72 | 1.66 | 0.15 | 0.12 | 0.03 |
| Example 2 | 3.41 | 3.44 | 0.06 | 0.03 | 0.03 |
| Example 3 | 4.38 | 4.30 | 0.15 | 0.07 | 0.08 |
| Example 4 | 6.17 | 5.45 | 1.47 | 0.67 | 0.8 |
| Example 5 | 6.74 | 6.14 | 4.62 | 2.03 | 2.59 |
| Example 6 | 7.49 | 6.63 | 6.18 | 2.67 | 3.51 |
| Example 7 | 7.95 | 7.04 | 5.71 | 2.47 | 3.24 |
| Example 8 | 9.27 | 7.06 | 5.88 | 2.56 | 3.32 |
| Example 9 | 10.66 | 7.17 | 6.04 | 2.82 | 3.22 |
| Example 10 | 6.08 | 3.93 | 0.07 | 0.04 | 0.03 |

### Test Example 2: Confirmation of whether or not API is dissolved, dissolution time and pH according to solvent composition (Example 11 to 20)

Solvents with the composition shown in Table 2 were prepared, for preparing 10 mg/ml solutions by dissolving the API. As shown in Table 2, the sodium phosphate buffer (PB) concentration of 0 to 10 mM, the NaOH concentration of 23 to 50 mM, the Tween^{®}80 concentration of 0 to 0.4 (w/v)%, and the hydroxypropyl beta cyclodextrin (HPβCD) concentration of 0 to 20(w/v)% was made as a solvent. Since the API has a characteristic of being soluble in basic solutions, solvents of pH 11 to 12 were prepared using NaOH and/or PB.

The sodium phosphate buffer was prepared at pH 7.4 by mixing sodium phosphate dibasic heptahydrate and sodium phosphate monobasic monohydrate. For HPβCD, a parenteral grade reagent with a molar substitution range of 0.81 to 0.99 and a molecular weight of about 1500 g/mol was used.

**[Table 2]**

| Example | Solvent Composition | Solvent pH |
|---|---|---|
| Example 11 | 50 mM NaOH | 12.4 |
| Example 12 | 30 mM NaOH | 12.1 |
| Example 13 | 25 mM NaOH | 12.1 |
| Example 14 | 25 mM NaOH | 12.0 |
| | 0.4(w/v)% Tween^{®} 80 | |
| Example 15 | 25 mM NaOH | 11.0 |
| | 20(w/v)% HPβCD | |
| Example 16 | 10 mM PB pH 7.4 | 12.3 |
| | 50 mM NaOH | |
| | 0.4(w/v)% Tween^{®} 80 | |
| Example 17 | 10 mM PB pH 7.4 | 11.8 |
| | 30 mM NaOH | |
| | 0.4(w/v)% Tween^{®} 80 | |
| Example 18 | 10 mM PB pH 7.4 | 11.8 |
| | 27 mM NaOH | |
| | 0.4(w/v)% Tween^{®} 80 | |
| Example 19 | 10 mM PB pH 7.4 | 11.7 |
| | 25 mM NaOH | |
| | 0.4(w/v)% Tween^{®} 80 | |
| Example 20 | 10 mM PB pH 7.4 | 11.6 |
| | 23 mM NaOH | |
| | 0.4(w/v)% Tween^{®} 80 | |

After putting the solvent (1 ml) prepared by Examples 11 to 20 according to the composition into a container with a lid, the API was added thereto at a concentration of 10 mg/ml, the container was capped and was shaken by hands for up to 10 minutes to mix. At this time, whether the API was dissolved or not, the time required for the API to be completely dissolved (dissolution time), and the pH of the solvent after dissolving the API were measured and shown in Table 3.

The solvents prepared in Examples 11 to 13 contained 25 to 50 mM NaOH, and no other excipients were used. Example 1 with NaOH concentration of 50 mM and Example 2 with 30 mM NaOH dissolved 10 mg/ml of API well, but since the pH of the API-dissolved solution is over 10, the formulation may cause pain to the patient when administered into the joint cavity. Therefore, Examples 11 and 12 are not suitable solvents for dissolving the API. In addition, Example 13 with a NaOH concentration of 25 mM could not be a suitable solvent because it did not completely dissolve the API. Therefore, from the results of Examples 11 to 13, it can be seen that a solvent containing only NaOH is not suitable as a solvent for dissolving the API.

Examples 14 and 15 are solvents prepared by including polysorbate 80 (hereinafter referred to as "Tween^{®} 80") or HPβCD as a solubilizing agent in 25 mM NaOH, respectively, and the API was not partially dissolved in each solvent. Accordingly, it can be seen that a solvent further comprising a solubilizing agent in NaOH is also not suitable as a solvent for dissolving the API.

The solvents prepared in Examples 16 to 20 were the same including 0.4 (w/v)% Tween^{®} 80 in 10 mM PB, and only the content of NaOH was different, but the API dissolved well in all solvents. However, in Examples 16 and 17, the pH was still high at 11.3 and 9.4, respectively, after the API was dissolved, whereas in Examples 18 to 20, the pH was 7.0 to 7.3, so it can be seen that the solvent is suitable for use in a preparation for injection.

In addition, Example 19 only additionally included 10 mM PB in the composition of Example 14, but the undissolved 10 mg/ml API was dissolved, and the pH of the solution after dissolution was properly adjusted to 7.3. Therefore, in selecting a solvent for dissolving the API of the present invention, it can be seen that the presence of PB that acts as a pH buffer is very important.

HPβCD surfactant did not show a significant effect compared to 0.4% Tween^{®} 80 even at 20%, and the osmotic pressure could be too high when the HPβCD content increased, so Tween^{®} 80 was selected as a surfactant.

**[Table 3]**

| Example | Dissolution of API 10mg/ml | Time to dissolve 100% | pH measured after mixing with API |
|---|---|---|---|
| Example 11 | ○ | 0.5 min | 11.7 |
| Example 12 | ○ | 2 min | 10.4 |
| Example 13 | Not completely dissolved | - | 8.5 |
| Example 14 | Not completely dissolved | - | 8.0 |
| Example 15 | Not completely dissolved | - | 7.8 |
| Example 16 | ○ | 0.5 min | 11.3 |
| Example 17 | ○ | 4 min | 9.4 |
| Example 18 | ○ | 5 min | 7.2 |
| Example 19 | ○ | 5 min | 7.3 |
| Example 20 | ○ | 7 to 10 min | 7.0 |

| | | | |
|---|---|---|---|
| (O: API is completely dissolved and the solution is transparent) | | | |

### Test Example 3: Confirmation of osmotic pressure according to solvent composition (Example 21 to 25)

Solvents of Examples 21 to 25 were prepared with the compositions shown in Table 4. After measuring the osmotic pressure of each solvent using an osmometer, 10 mg/ml API was dissolved and the osmotic pressure of the solution (mixture) was measured.

**[Table 4]**

| Example | Solvent Composition | Solvent pH |
|---|---|---|
| Example 21 | 50 mM PB | 7.3 |
| | 25 mM NaOH | |
| | 0.4(w/v)% Tween^{®} 80 | |
| Example 22 | 100 mM PB | 7.3 |
| | 25 mM NaOH | |
| | 0.4(w/v)% Tween^{®} 80 | |
| Example 23 | 130 mM PB | 7.3 |
| | 25 mM NaOH | |
| | 0.4(w/v)% Tween^{®} 80 | |
| Example 24 | 10 mM PB | 11.7 |
| | 25 mM NaOH | |
| | 0.4(w/v)% Tween^{®} 80 | |
| | 0.6(w/v)% NaCl | |
| Example 25 | 10 mM PB | 11.7 |
| | 25 mM NaOH | |
| | 0.4(w/v)% Tween^{®} 80 | |
| | 0.7(w/v)% NaCl | |

All of the solvents in Examples 21 to 25 dissolved 10 mg/ml API well, and the osmotic pressure of the solvent, the osmotic pressure of the solution in which the API was dissolved, and the difference between the osmotic pressures are shown in Table 5.

In all of Examples 21 to 25, the osmotic pressure increased after dissolving 10 mg/ml compared to the solvent osmotic pressure. In particular, in the case of Examples 21 to 23 in which the osmotic pressure was increased with PB, the osmotic pressure difference was 22 to 23 mOsm/kg. Since the molarity is 24 mM when 10 mg/ml of the API is dissolved, it can be seen that the API is completely dissolved in Examples 21 to 23. However, in Examples 24 and 25, in which the osmotic pressure of the solvent was adjusted with NaCl, the API was not completely dissolved, and the osmotic pressure difference was 11 to 13 mOsm/kg, reflecting this.

In addition, Examples 24 and 25 are solvents with a higher pH than Examples 21 to 23, but did not completely dissolve the API. From this, it can be seen that a certain level of buffering capacity is essential to dissolve the API of the present invention.

In addition, the preferred osmotic pressure range of the formulation for use as an injection is 280 to 330 mOsm/kg, and patients may feel pain if it falls below or exceeds this range. Therefore, when these criteria are applied, it can be seen that the formulation of Example 23 having a final solution osmotic pressure of 318 mOsm/kg is preferable as an injection formulation.

**[Table 5]**

| Example | Dissolution of API 10mg/ml | Solvent Osmotic pressure (mOsm/Kg) | Solution Osmotic pressure (mOsm/Kg) | Osmotic pressure difference (mOsm/Kg) |
|---|---|---|---|---|
| Example 21 | ○ | 129 | 152 | 23 |
| Example 22 | ○ | 236 | 259 | 23 |
| Example 23 | ○ | 296 | 318 | 22 |
| Example 24 | Δ | 249 | 262 | 13 |
| Example 25 | Δ | 277 | 288 | 11 |

| | | | | |
|---|---|---|---|---|
| (O: API is completely dissolved and the solution is transparent, △: Part of API is not dissolved) | | | | |

### Test Example 4: Confirmation of API stability in solution

API (Active form) of Formula 1 is rapidly hydrolyzed in aqueous solution and turns into an inactive. Therefore, in order to measure the content of the active form in the solution after preparing the injection, 10 mg/ml of API was dissolved using the solvent of Example 23, and the concentration and purity of the active API (Compound of Formula 2), which changed over time, were measured using HPLC until 60 minutes after dissolution.

As shown in Table 6 below, after preparing an API solution by mixing 10 mg/ml API with the solvent of Example 23, the combined concentration of the compounds of Formula 2 (referred to as 'Active API') and Formula 3 (hereinafter referred to as 'Inactive API') (the combined amount of Active and Inactive APIs) reached a concentration of 9.69 mg/ml by 10 minutes after mixing and maintained a similar concentration until 30 minutes, but showed a tendency to decrease after 60 minutes after mixing.

However, in the case of the active API, it continued to decrease after mixing. After 30 minutes of mixing, the active API content was about 95.5% and the inactive API content was 4.33%, whereas after 60 minutes, the active API content was 94.18% and the inactive API content was 5.59%. Therefore, it was confirmed that the API of the present invention should be used within 30 minutes after solvent mixing because it does not reach the active API content of 95% or more, which is a normal API specification.

**[Table 6]**

| Measurement time after mixing (min) | Concentration (mg/ml) | | | Peak Area(%) | |
|---|---|---|---|---|---|
| | Total | Active | Inactive | Active | Inactive |
| 3.5 | 9.02 | 8.76 | 0.26 | 97.12 | 2.84 |
| 5 | 9.3 | 9.03 | 0.27 | 97.08 | 2.85 |
| 10 | 9.69 | 9.4 | 0.29 | 96.92 | 3.00 |
| 30 | 9.73 | 9.31 | 0.42 | 95.52 | 4.33 |
| 60 | 9.46 | 8.93 | 0.53 | 94.18 | 5.59 |

### Test Example 5: Preparation of API solutions with various contents

As shown in Table 7, based on the solvent of Example 23, the solvents of Examples 26 to 28 were prepared by changing the composition of PB and NaOH in consideration of the solubility of the API of the present invention at basic pH and the osmotic pressure of the final solution.

**[Table 7]**

| Example | API Content | Solvent Composition |
|---|---|---|
| Example 26 | 2 mg/ml | 130 mM PB |
| | | 5 mM NaOH |
| | | 0.4 (w/v)% Tween^{®} 80 |
| Example 27 | 5 mg/ml | 120 mM PB |
| | | 12.5 mM NaOH |
| | | 0.4 (w/v)% Tween^{®} 80 |
| Example 28 | 15 mg/ml | 110 mM PB |
| | | 37.5 mM NaOH |
| | | 0.4 (w/v)% Tween^{®} 80 |

After measuring the pH and osmotic pressure of the solvents in Examples 26 to 28, 2 mg/ml, 5 mg/ml and 15 mg/ml API were dissolved, respectively, and then the pH and osmotic pressure of the mixed solution were measured. As a result, as shown in Table 8, it can be seen that when the API is dissolved using the solvents of Examples 26 to 28, the pH and osmotic pressure of the mixed solution are both suitable for administration into the patient's joint cavity.

**[Table 8]**

| Example | API Content (mg/ml) | Solvent | | Solution | |
|---|---|---|---|---|---|
| | | pH | Osmotic pressure (mOsm/Kg) | pH | Osmotic pressure (mOsm/Kg) |
| Example 26 | 2 | 7.34 | 293 | 7.29 | 297 |
| Example 27 | 5 | 7.57 | 277 | 7.3 | 290 |
| Example 28 | 15 | 10.65 | 266 | 7.31 | 299 |

Next, the API concentration and purity of the injections prepared with the solvents of Examples 26 to 28 were measured immediately after preparation, after 30 minutes, and after 60 minutes, respectively, and the results are shown in Table 9. The purity immediately after mixing was measured to be about 97%, the purity after 30 minutes of mixing was measured to be 95 to 96%, and the purity after 60 minutes of mixing was measured to be 93 to 94%. Therefore, in the finished product of all doses, appropriate purity is maintained until 30 minutes after mixing, and the inactive API is produced beyond the standard (Inactive API is less than 5%) after 60 minutes of mixing, so it is not suitable for administration to patients. Thus, it was confirmed that all finished products should be prepared immediately prior to use and injected within 30 minutes after mixing.

**[Table 9]**

| Example | API Content (mg/ml) | Immediately after dissolution | | After 30 min | | After 60 min | |
|---|---|---|---|---|---|---|---|
| | | Active (%) | Inactive (%) | Active (%) | Inactive (%) | Active (%) | Inactive (%) |
| Example 26 | 2 | 97.09 | 2.91 | 95.98 | 3.93 | 94.25 | 5.52 |
| Example 27 | 5 | 97.23 | 2.77 | 95.83 | 4.04 | 94.29 | 5.48 |
| Example 28 | 15 | 96.88 | 3.06 | 95.63 | 4.23 | 93.85 | 5.9 |

## Claims

1. A preparation for injection, comprising:
a first formulation containing the compound of Formula 1: 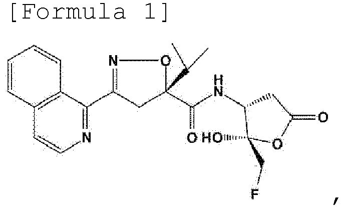 and
a second formulation, containing a phosphate buffer to dissolve the compound of Formula 1, surfactant, and NaOH as a pH adjusting agent, and the second formulation has a pH of 7 to 11.

2. The preparation for injection according to claim 1, wherein the first formulation is in powder form.

3. The preparation for injection according to claim 1, wherein the compound of Formula 1 is contained in an amount of 5 to 20 mg/ml based on a total volume of a mixed solution of the first formulation and the second formulation.

4. The preparation for injection according to claim 1, wherein a mixed solution of the first formulation and the second formulation has a pH of 6 to 8.

5. The preparation for injection according to claim 1, wherein a mixed solution of the first formulation and the second formulation has an osmotic pressure of 280 to 330 mOsm/kg.

6. The preparation for injection according to claim 1, wherein the phosphate buffer is a mixture of sodium phosphate dibasic heptahydrate and sodium phosphate monobasic monohydrate.

7. The preparation for injection according to claim 6, wherein the phosphate buffer is contained in an amount of 100 to 130 mM based on a total volume of the second formulation.

8. The preparation for injection according to claim 1, wherein the surfactant is selected from polysorbate 20, polysorbate 40, polysorbate 60, polysorbate 65, and polysorbate 80.

9. The preparation for injection according to claim 8, wherein the surfactant is polysorbate 80.

10. The preparation for injection according to claim 9, wherein the surfactant is contained in an amount of 0.2 to 5.0(w/v)% based on a total volume of the second formulation.

11. The preparation for injection according to claim 1, wherein the NaOH is contained in an amount of 12 to 42 mM based on a total volume of the second formulation.

12. The preparation for injection according to claim 1, wherein the preparation for injection is for treating or preventing a disease selected from osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes, and gastric ulcer.

13. The preparation for injection according to claim 12, wherein the disease is selected from osteoarthritis, rheumatoid arthritis, degenerative arthritis and destructive bone disorder.

14. The preparation for injection according to claim 1, wherein a mixed solution of the first formulation and the second formulation is for a single-dose injection.

15. The preparation for injection according to claim 14, wherein the preparation for injection is administered into the joint cavity after the first formulation and the second formulation are mixed.

16. The preparation for injection according to claim 15, wherein the preparation for injection is administered into the joint cavity within 30 minutes after the first formulation and the second formulation are mixed.

17. The preparation for injection according to claim 16, wherein the preparation for injection is administered once after the first formulation and the second formulation are mixed.

18. An preparation for injection comprising:
a first formulation containing the compound of Formula 1: 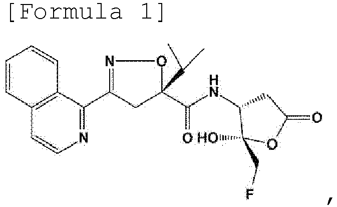 and
a second formulation having a pH of 7 to 11 buffer for dissolving the compound of Formula 1,
wherein, a content of the compound Formula 2 is at least 95% based on a total content of the compounds of Formula 2 and Formula 3, as measured within 30 minutes after the first formulation and the second formulation are mixed.
